# EUROPEAN PATENT SPECIFICATION

(11) **EP 0 336 877 B1**
(45) Date of publication and mention of the grant of the patent: **27.10.1993**
(21) Application number: 89730025.7
(22) Date of filing: 06.02.1989
(51) Int. Cl.: A61F 2/16

(54) **Intraocular lens**
Intraokulare Linse
Lentille intra-oculaire

(30) Priority: 08.02.1988 US 153159
(43) Date of publication of application: 11.10.1989
(73) Proprietor: Herman, Wesley K., Dallas Texas 75231 (US)
(72) Inventor: Herman, Wesley K., Dallas Texas 75231 (US)
(74) Representative: UEXKÜLL & STOLBERG

(56) References cited:
- EP-A- 0 125 361
- EP-A- 0 178 049
- US-A- 4 661 108
- US-A- 4 701 181

## Description

### TECHNICAL FIELD

This invention relates to an intraocular lens intended for implantation in the posterior chamber of the eye following extracapsular cataract extraction, and, in particular, to an intraocular lens having a haptic constructed to avoid lateral movement of the lens during ciliary muscle contraction, while providing anterior or posterior displacement of the intraocular lens.

### BACKGROUND ART

A cataract is a cloudiness or opacity which develops in the lens of the eye which is normally clear and transparent. A person with impaired vision due to a cataract may have his vision improved through a combination of cataract surgery and proper corrective lenses.

An ophthalmologic surgeon may elect one of several different surgical procedures for removing a lens that has a cataract. Intracapsular cataract extraction is a technique for the removal of the entire cataract, including its capsule, in one piece. Extracapsular cataract extraction and phacoemulsification and aspiration are two surgical techniques that involve the removal of the opacified portions of the lens, while the clear posterior capsule which was the original support for the lens is left in place.

Intracapsular or extracapsular extraction eliminates the cloudiness or opacity caused by the cataract, but light entering the eye is now unfocused since the lens has been removed. Light entering the eye may be focused by an intraocular lens.

Ophthalmologic surgeons now generally recognize that it is better optically and physiologically to implant the intraocular lens into the posterior chamber of the eye. Intraocular lens implants have also been done in the anterior chamber in which the lens is implanted forward of or mounted to the iris. Implantation of the intraocular lens behind the pupil has been found to cause fewer secondary problems following extracapsular surgery, particularly glaucoma and swelling of the eye called cystoid macular edema.

Intraocular lens implants into the posterior chamber also have been found to have some secondary complications. Nearly half of the patients who have had extracapsular cataract extraction experience some loss of visual function from clouding of the posterior capsule within several months to several years after the initial surgery. In order to restore a patient's visual function, further surgery becomes necessary to make an opening in the posterior capsule with a knife or a laser.

Ophthalmologic surgeons in the past have tried to design intraocular lenses for implantation into the posterior chamber that retard the opacification or clouding of the posterior capsular membrane left following the extracapsular cataract extraction. One of the first intraocular posterior chamber lenses designed to retard opacification and to facilitate discission of the membrane is diclosed in U.S. Patent No. Re. 31,626, issued on July 10, 1984 to Kenneth J. Hoffer. Hoffer designed a lens having a convex-plano relationship where the power is put in the front or anterior surface of the lens. The rear or posterior surface has a ridge intended to prevent the lens cells from sliding in beneath the ridge, thus retarding opacification. The Hoffer lens had an opening along the circumferential ridge for insertion of a surgical knife or needle through the opening in the posterior chamber in the space behind the lens, but there is no intended contact of the lens optic with the posterior capsular membrane.

Another design for an intraocular posterior chamber lens is disclosed in US-A-4,485,499, issuing to Lawrence D. Castleman. The lens has a convex front face and a generally planar rear face with two parallel projecting members. The projecting members were said to provide a separation or open zone at the visual axis between the lens body and posterior capsule to facilitate corrective discission surgery. Again, this lens does not provide contact of the lens optic with the posterior capsular membrane (PCM).

Yet another design of a posterior intraocular lens is disclosed in U.S. Patent Re. 31,998, reissued on October 8, 1985 to William D. Myers. The Myers lens implant was intended to facilitate laser posterior capsulotomy following extracapsular surgery. In one form, the lens has a front convex surface, and a bridge spaces the generally planar rear surface of the optic forward of the posterior capsule. In a second form, the lens has a convex front surface, but the rear surface optic is concave to provide a space forward of the posterior capsule. This lens also does not provide lens optic contact with the PCM.

US-A-4 661 108 which has been used for the delineation of Claim 1 shows an intraocular lens body having a haptic extending therefrom, said haptic having a smooth, unsegmented design.

It is one object of the present invention to provide an improved implantable intraocular lens having a haptic design that will minimize lateral movement of the lens and other potentially traumatizing effects of ciliary muscle contraction upon the intraocular lens, while utilizing the force exerted by the ciliary muscle upon the haptic to displace the optic anteriorally or posteriorly.

### BACKGROUND OF THE INVENTION

The invention relates to an intraocular lens including a lens body and haptics attached to and extending from said lens body for implantation in the posterior chamber of an eye following extracapsular cataract extraction,
**characterized by:**
each said haptics having a proximal portion extending anteriorly from said lens body, and a distal portion extending from said proximal portion, said distal portion being constructed to lie substantially parallel to a plane defined by the iris of the eye when said intraocular lens is implanted in the posterior chamber of the eye.

The lens is designed to maintain a constant relationship with the posterior capsule and the dioptric power of the lens is fixed posteriorly for all dioptric ranges. Power modification to the lens is achieved by modifications of the front or anterior surface of the lens. The posterior surface of the lens contacts the posterior capsule over a constant area of the posterior capsule to provide improved early visual acuity and to retard opacification by blocking migration of epithelial cells between the posterior capsule and the lens. A circumferential edge portion of the lens is angled posteriorly to contact and support the edge of the lens on the surface of the posterior capsule. The angled circumferential edge of the lens in combination with the design of the convex rear surface creates an annular space between the lens and the posterior capsule to facilitate any discission of the membrane with a laser or knife. The circumferential edge also serves to block migration of cells beneath the lens. Multisectional or gull-winged haptics are attached to the edge of the lens for securing it within the posterior capsule such that only a minimal amount of lateral movement of the lens results from contraction of the ciliary muscle.

### BRIEF DESCRIPTION OF THE DRAWINGS

A more complete understanding of the present invention and its advantages will be apparent from the following Detailed Description taken in conjunction with the accompanying Drawings in which:
FIG. 1 is a top plan view of one embodiment of the posterior intraocular lens of the present invention;
FIG. 2A is a side elevation view of the lens shown in FIG. 1 with a gull-winged haptic.
FIG. 2B is an alternate embodiment of the lens shown in FIG. 2A in which the lens has a different power modification.
FIG. 2C is an alternate embodiment of the lens shown in FIG. 2A in which the lens has a different power modification;
FIG. 3 is a cross sectional schematic view of a portion of the human eye showing a lens of the present invention having a gull-winged haptic implanted following a capsular cataract extraction.
FIG. 4 is a side elevation view of the lens shown in FIG. 1 with a compound gull-winged haptic.

### DETAILED DESCRIPTION

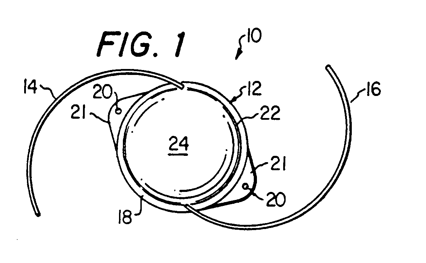

FIG. 1 illustrates a top plan view of the posterior chamber intraocular lens of the present invention, generally identified by the reference numeral 10. The intraocular lens 10 has a lens body 12 with associated haptic members 14 and 16 attached to the periphery of the lens body 12. Manipulation holes 20 and tabs 21 are not essential to the present invention but may be included to provide the ophthalmologic surgeon with a means to facilitate placement of the lens 12 in the human eye. An edge region 18 is angled posteriorly for lifting the lens body 12 off the posterior capsule in the region outside of the optical zone of the eye. The edge region 18 begins along the circumlinear line 22 where the anterior surface 24 begins to angle posteriorly.

The lens body 12 of the lens 10 would typically be approximately 6 millimeters in diameter. The manipulation holes 20 may be on the order of 0.4 millimeter in diameter.

The lens body 12 of the posterior chamber intraocular lens 10 may be made from polymethylmethacrylate (PMMA). The optic material for the lens body 12 may be made with any other suitable material to create the necessary optics for the patient, but the material selected should be one that is acceptable for use with laser surgery, such as a yttrium aluminum garnet (YAG) laser, for secondary cataract procedures. In addition, the lens body 12 may be made from a material such as silicone or an acrylic for use as a soft injectable intraocular lens. The optic design of the intraocular lens 10 and associated haptic design may be particularly suitable for use in a soft injectable lens design.

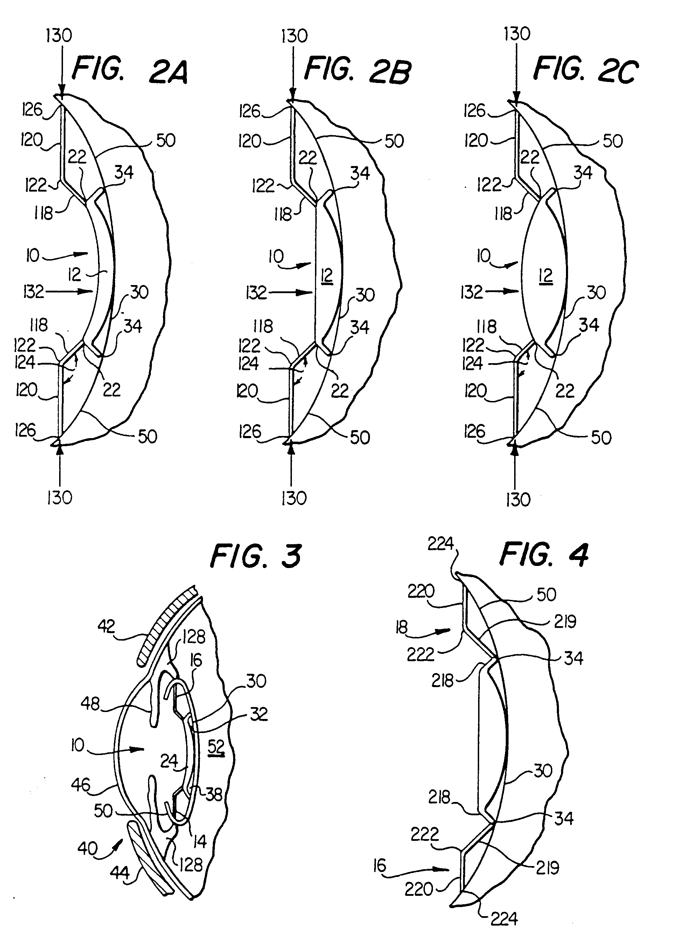

The lens body 12 of intraocular lens 10 may be manufactured from any of the four principal technologies used for manufacturing intraocular lenses. First, in injection molding, plastic is injected into a mold design for the lens in a liquid state at very high pressure, permitting removal of the lens when it is in the solid state. The injection molding technique is one of the oldest and could be used in manufacturing the lens 10 of the present invention. However, a lens made with this technique may be more susceptible to damage from a YAG laser than lenses made from other technologies. In cast molding technology, a lens is made from two halves that are assembled together to form the optic. The lens 10 of the present invention would be particularly suitable for this technique since one half of the cast would have a fixed design, the posterior surface of the lens body 12. The anterior or front half of the lens body 10 could be made to provide any of the various power modifications required for the lens 10. The anterior surface or front half of the lens body could be either concave, plano or convex, as illustrated and further described in FIGS. 2A-C below. In cast molding or injection molding, approximately 20 or 30 front halves could be utilized to cover the desired optic powers for the lens. In lathe technology, the optic or lens body 12 could be cut from a single piece of material, and haptics could be simultaneously cut or inserted into the optic. Individual optic designs could be accomplished by inputting the power requirements on the anterior surface into a computer controlling the lathe operation. The specification of the posterior surface would be constant for all lens powers. Finally, the lens 10 could be made from silicone, acrylic, or any other suitable pliable material. Lens 10 made from a pliable material would be particularly suitable for use as a lens injected into the eye through a small incision.

As best seen in FIGS. 2A-C and 3, haptics 14, 16 include proximal sections 118 and distal sections 120. Proximal sections 118 extend from circumlinear line 22 of intraocular lens 10. Proximal sections 118 are upwardly inclined relative to intraocular lens 10 such that they extend toward iris 48. Proximal sections 118 terminate at their point of intersection 122 with distal sections 120. Distal sections 120 are disposed in substantially parallel relation to iris 48. Displacement angle 124 between proximal sections 118 and distal sections 120 is preferably obtuse for reasons discussed in detail below. It is to be appreciated that haptics 14, 16 may have any form, eg. C-shaped, J-shaped, etc., and may include eyelets, positioning notches, or closed loops, so long as an obtuse displacement angle 124 is formed between proximal sections 118 and distal sections 120.

In use, terminal ends 126 of distal sections 120 are in contact with lens capsule 50 posterior to iris 48. Contraction of ciliary muscles 128 creates a force in the direction of arrows 130 on distal sections 120. The force thus created is transferred from distal sections 120 to proximal sections 118 and finally to lens 12. The resulting force on lens 12 has a first force vector 132 directed towards and perpendicular to posterior capsular membrane 30, causing lens 12 to be urged posteriorly. Because ciliary muscle contraction is substantially symmetric, there is little or no resulting force on lens 12 in a direction perpendicular to vector 132. Thus, lens 12 remains centered on posterior capsular membrane 30 during ciliary muscle contraction. This characteristic is particularly desirable to prevent stretching or folding of posterior capsular membrane 30 which would result from lateral movement of lens 12. Lateral movement of an intraocular lens is known to cause multiple imaging and reduced visual acuity due to the resulting nonuniform stretching of the posterior capsular membrane. Thus, haptics 14, 16 decrease both the incidence of lateral movement of lens 12 and the incidence of lost visual acuity due to posterior capsular irregularities. Haptics 14, 16 are also less likely, relative to known haptics, to puncture the sulcus or zonules during ciliary muscle contraction. Such puncturing is known to cause complications such as iritis, cystoid macular edema, and hemorrhaging.

When the ciliary muscle is relaxed, lens 12 will tend to be displaced anteriorly relative to its position during ciliary muscle contraction. Anterior displacement increases the vergence of intraocular lens 12, causing it to focus from one to three diopters closer. This characteristic would permit an emmetropic pseudophake to read upon relaxation of the ciliary muscle. As a corollary, posterior displacement of lens 12 decreases its vergence power permitting a myopic pseudophake to approach emmetropia upon contraction of the ciliary muscle. Such contraction of the ciliary muscle can be achieved naturally or it can be chemically induced.

In a second preferred embodiment depicted in FIG. 4, haptics 14, 16 have proximal portions 219, intermediate portions 218, and distal portions 220. Intermediate portions 218 extend posteriorly from lens body 12 towards posterior capsular member 30. Proximal portions 219 intersect intermediate portions 218 at points 34. It is to be appreciated that intermediate portions 218 can be replaced by edge regions 18 without changing the character of this second preferred embodiment.

Points 34 are in contact with posterior capsular membrane 30 and therefore tend to elevate portions of lens body 12 relative to posterior capsular membrane 30. Proximal portions 219 extend anteriorly from points 34 and terminate at intersection points 222. Distal portions 220 extend radially outward from intersection points 222 in substantially parallel relation to iris 48. Distal portions 220 terminate at ends 224. Ends 224 are in contact with posterior capsule 50.

In use, contraction of the ciliary muscle causes lens 12 to be displaced anteriorly due to the configuration of haptics 16, 18 in the second preferred embodiment. That is, contraction of the ciliary muscle causes a greater force to be directed perpendicularly to posterior capsular membrane 30 at points 34, causing lens body 12 to be displaced anteriorly. Relaxation of the ciliary muscles will cause lens body 12 to be displaced posteriorly relative to its position during contraction of the ciliary muscle. Thus, in this second embodiment, the effects of ciliary muscle contraction and relaxation upon the vergence of lens body 12 is opposite to the effects discussed above with respect to the first preferred embodiment of haptics 16, 18.

The haptics 14, 16 may be made out of polypropylene, Prolene (a trademark of Ethicon, Inc.), extruded PMMA, polyamide, silicone, acrylic, or any other material having comparable characteristics. Such materials would be suitable for use with pliable lenses to make an injectable lens 10.

## Claims

1. An intraocular lens (10) including a lens body (12) and haptics (14, 16) attached to and extending from said lens body (12) for implantation in the posterior chamber of an eye (40) following extracapsular cataract extraction,
**characterized by:**
each of said haptics (14, 16) having a proximal portion (118, 219) extending anteriorly from said lens body (12), and a distal portion (120, 220) extending from said proximal portion (118, 219), said distal portion (120, 220) being constructed to lie substantially parallel to a plane defined by the iris (48) of the eye (40) when said intraocular lens (10) is implanted in the posterior chamber of the eye.

2. The intraocular lens of claim 1, wherein the proximal portion (219) connects to an intermediate portion (218) which extends posteriorly from said lens body (12).

3. The intraocular lens of claim 1 or 2, wherein said distal portions (120, 220) of said haptics (14, 16) extend from said proximal portions (118, 219) at a predetermined obtuse angle.

4. The intraocular lens of claim 1 or 2, wherein said intermediate portions (218) of said haptics (14, 16) extend posteriorly from the entire circumference of said lens body (12).

5. The intraocular lens of claim 1 or 2, wherein each said haptic (14, 16) is substantially J-shaped.

6. The intraocular lens of claim 1 or 2, wherein eyelets are positioned on said distal portions (120, 220) of said haptics (14, 16).

7. The intraocular lens of claim 1 or 2, wherein positioning notches (20) are formed on said distal portions (120) of said haptics (14).

8. The intraocular lens of claim 1 or 2, wherein said haptics (14, 16) have a closed loop configuration.

## Patentansprüche

1. Intraokulare Linse (10) mit einem Linsenkörper (12) und Scleralschalen (14, 16), die sich zum und vom Linsenkörper (12) erstrecken, für die Implantation in der hinteren Kammer eines Auges (40) nach einer extracapsularen Operation des grauen Stars, **dadurch gekennzeichnet,** daß
jede der Scleralschalen (14, 16) einen proximalen Teil (118, 219), der sich vom Linsenkörper (12) nach vorn erstreckt und einen distalen Teil (120, 220), der sich vom proximalen Teil (118, 219) erstreckt, aufweist und der distale Teil (120, 220) so aufgebaut ist, daß er im wesentlichen parallel zu einer durch die Iris (48) des Auges (40) definierten Ebene verläuft, wenn die intraokulare Linse (10) in der hinteren Kammer des Auges implantiert ist.

2. Intraokulare Linse nach Anpruch 1, in der der proximale Teil (219) mit einem Zwischenteil (218) verbunden ist, der sich vom Linsenkörper (12) nach hinten erstreckt.

3. Intraokulare Linse nach Anspruch 1 oder 2, in der die distalen Teile (120, 220) der Scleralschalen (14, 16) sich von den proximalen Teilen (118, 219) unter einem vorher festgelegten stumpfen Winkel erstrecken.

4. Intraokulare Linse nach Anspruch 1 oder 2, in der die Zwischenteile (218) der Scleralschalen (14, 16) sich vom gesamten Umfang des Linsenkörpers (12) nach hinten erstrekken.

5. Intraokulare Linse nach Anspruch 1 oder 2, in der jede der Scleralschalen (14, 16) im wesentlichen J-förmig ausgebildet ist.

6. Intraokulare Linse nach Anspruch 1 oder 2, in der die Löcher auf den distalen Teilen (120, 220) der Scleralschalen (14,16) angeordnet sind.

7. Intraokulare Linse nach Anspruch 1 oder 2, in der Positionierschlitze (20) auf den distalen Teilen (120) der Scleralschalen (14) ausgebildet sind.

8. Intraokulare Linse nach Anspruch 1 oder 2, in der die Scleralschalen (14, 16) in einer geschlossenen Schleife ausgeführt sind.

## Revendications

1. Lentille intra-oculaire (10) comprenant un corps de lentille (12) et des haptiques (14, 16) fixées audit corps de lentille (12) et s'étendant à partir de celui-ci, pour l'implantation dans la chambre postérieure d'un oeil (40) après une extraction de cataracte extracapsulaire,
caractérisée en ce que :
chacune desdites haptiques (14, 16) a une partie proximale (118, 219) s'étendant antérieurement à partir dudit corps de lentille (12), et une partie distale (120, 220) s'étendant à partir de ladite partie proximale (118, 219), ladite partie distale (120, 220) étant réalisée pour être sensiblement parallèle à un plan défini par l'iris (48) de l'oeil (40) lorsque ladite lentille intra-oculaire (10) est implantée dans la chambre postérieure de l'oeil.

2. Lentille intra-oculaire selon la revendication 1, dans laquelle la partie proximale (219) est reliée à une partie intermédiaire (218) qui s'étend postérieurement à partir dudit corps de lentille (12).

3. Lentille intra-oculaire selon la revendication 1 ou 2, dans laquelle lesdites parties distales (120, 220) desdites haptiques (14, 16) s'étendent à partir desdites parties proximales (118, 219) selon un angle obtus prédéterminé.

4. Lentille intra-oculaire selon la revendication 1 ou 2, dans laquelle lesdites parties intermédiaires (218) desdites haptiques (14, 16) s'étendent postérieurement à partir de toute la circonférence dudit corps de lentille (12).

5. Lentille intra-oculaire selon la revendication 1 ou 2, dans laquelle chacune desdites haptiques (14, 16) a sensiblement la forme d'un J.

6. Lentille intra-oculaire selon la revendication 1 ou 2, dans laquelle des oeillets sont disposés sur lesdites parties distales (120, 220) desdites haptiques (14, 16).

7. Lentille intra-oculaire selon la revendication 1 ou 2, dans laquelle des encoches de positionnement (20) sont formées sur lesdites parties distales (120) desdites haptiques (14).

8. Lentille intra-oculaire selon la revendication 1 ou 2, dans laquelle lesdites haptiques (14, 16) ont une configuration en boucle fermée.
